# EUROPEAN PATENT SPECIFICATION

(11) **EP 4 196 051 B1**
(45) Date of publication and mention of the grant of the patent: **20.05.2026**
(21) Application number: 21762987.2
(22) Date of filing: 10.08.2021
(51) Int. Cl.: A61F 2/24

(54) **EXPANDABLE ANNULOPLASTY RINGS**
EXPANDIERBARE ANNULOPLASTIERINGE
ANNEAUX D'ANNULOPLASTIE EXPANSIBLES

(30) Priority: 11.08.2020 US 202063064287 P
(43) Date of publication of application: 21.06.2023
(73) Proprietor: Edwards Lifesciences Corporation, Irvine, CA 92614-5688 (US)
(72) Inventor: CAMPBELL, Louis, A., Irvine, CA 92614 (US); RODRIGUEZ, Rodolfo, Irvine, CA 92614 (US); CHANG, Da-Yu, Irvine, CA 92614 (US); BAK-BOYCHUK, Gregory, San Clemente, CA 92673 (US); CONKLIN, Brian, S., Orange, CA 92867 (US); MUNNELLY, Amy, E., Irvine, CA 92614 (US)
(74) Representative: Eisenführ Speiser
(86) International application number: PCT/US2021/071152
(87) International publication number: WO 2022/036358

(56) References cited:
- WO-A1-2010/014671
- WO-A1-2016/200993
- WO-A1-2020/028218
- US-A1- 2018 042 723

## Description

### TECHNICAL FIELD

The present disclosure relates generally to annuloplasty rings and, in particular, to expandable mitral annuloplasty rings.

### BACKGROUND

The heart is a hollow muscular organ having four pumping chambers separated by four heart valves: aortic, mitral (or bicuspid), tricuspid, and pulmonary. The mitral and tricuspid valves are defined by dense fibrous rings of collagen, each called an annulus, which forms a part of the fibrous skeleton of the heart. The annulus provides peripheral attachments for the two cusps or leaflets of the mitral valve (called the anterior and posterior cusps) and the three cusps or leaflets of the tricuspid valve. The native valve leaflets flex outward when the valve opens and their free edges come together or coapt in closure.

The free edges of the mitral leaflets connect to chordae tendineae from more than one papillary muscle. Mitral valve malfunction can result from the chordae tendineae (the chords) becoming stretched, and in some cases tearing. Also, a normally structured valve may not function properly because of an enlargement of or shape change in the valve annulus. This condition is referred to as a dilation of the annulus and generally results from heart muscle failure. In addition, the valve may be defective at birth or because of an acquired disease. From a number of etiologies, mitral valve dysfunction can occur when the leaflets do not coapt at peak contraction pressures. As a result, an undesired back flow of blood from the left ventricle into the left atrium can occur.

Various surgical techniques may be used to repair a diseased or damaged valve. A commonly used repair technique effective in treating incompetence is annuloplasty, which often involves reshaping or remodeling the annulus by attaching a prosthetic annuloplasty repair segment or ring thereto. For instance, the goal of a posterior mitral annulus repair is to bring the posterior mitral leaflet forward toward to the anterior leaflet to better allow coaptation. The annuloplasty ring is designed to support the functional changes that occur during the cardiac cycle: maintaining coaptation and valve integrity to prevent reverse flow while permitting good hemodynamics during forward flow.

Annuloplasty rings may be stiff, flexible or semi-rigid, and a "remodeling" annuloplasty ring typically has an inner core that resists conforming to the native annulus shape and instead forces the annulus to conform to it. Remodeling annuloplasty bands or rings are "generally rigid" or "semi-rigid" in that they will resist distortion when subjected to the physiological forces imparted thereon by the mitral valve annulus of an operating human heart. In this sense, "distortion" means substantial permanent deformation from a predetermined or manufactured shape (e.g., the band or ring will tend to return to its preset shape in use). A typical remodeling annuloplasty ring comprises an inner substrate or core of a metal such as a rod or multiple bands of nitinol, cobalt-chromium, stainless steel or titanium covered with a biocompatible fabric or cloth and perhaps silicone to allow the ring to be sutured to the fibrous annulus tissue.

Annuloplasty rings may have a variety of shapes in plan view, including closed or continuous oval, circular, D-shaped, or kidney-shaped, and open or discontinuous C-shaped, sometimes referred to as a band. Examples are seen in U.S. Patent Nos. 5,041,130, 5,104,407, 5,201,880, 5,258,021, 5,607,471 and, 6,187,040. Most rigid and semi-rigid annular rings for the mitral valve have a kidney-like or D shape, with a curved posterior segment co-extensive with the posterior valve leaflet, and a somewhat straighter anterior segment co-extensive with the anterior valve leaflet. One successful semi-rigid ring is the Carpentier-McCarthy-Adams IMR ETlogix^{®} annuloplasty ring sold by Edwards Lifesciences of Irvine, CA. The IMR ETlogix ring is semi-rigid for remodeling purposes, and is intended to support the P2/P3 segments of the mitral valve that have dilated because of ischemia.

Nearly 20,000 patients undergo mitral valve repair each year and approximately 60,000 go untreated in the US. Currently, approximately 30% of mitral repairs for ischemic functional mitral regurgitation (FMR) result in reoperations.

Sometimes the need for complete mitral valve replacement may arise after a patient has already had an earlier annuloplasty ring repair. Rather than explanting the annuloplasty ring, a new prosthetic heart valve may be expanded directly within the ring (a so-called valve-in-ring procedure). For instance, U.S. Patent No. 8,287,591 discloses various solutions for valve-in-ring systems. Clinical trials have been undertaken as well, such as described in "Transfemoral Tricuspid Valve-in-Ring Implantation Using the Edwards Sapien XT Valve," Circulation: Cardiovascular Interventions. 2015:8 (5 Mar 2015). Valve-in-valve procedures are somewhat more common, such as described in U.S. Patent No. 9,375,310.

WO 2016/200993 A1 describes an annuloplasty ring including elastic features for receiving a subsequent prosthetic valve via a "Valve In Ring Procedure." The elastic features are said to provide a squeezing force on the native valve annulus that both ensures coaptation of the native valve leaflets and also prevents paravalvular leakage around a subsequently-placed prosthetic valve.

US 2018/042723 Al is related to to a device for minimally invasive medical treatment. The device being a hollow tube with a first end, a second end, and one or more anchors configured to extend outward from the exterior of the hollow tube. The hollow tube having a plurality of cutouts on the exterior, wherein the cutouts allow the hollow tube to be flexible. Additionally, the hollow tube may have at least one snap mechanism configured to connect the first end and the second end together.

Despite some research into valve-in-ring and valve-in-valve procedures, there is a need for an annuloplasty ring that better accommodates a subsequently implanted expandable valve.

### SUMMARY

The present invention is as set forth in the appended claims. The present disclosure provides a mitral repair annuloplasty ring that will accommodate implantation of a transcatheter valve when there is a need for a reoperation of a patient that may not undergo open heart surgery. The mitral repair ring is a rigid/semi-rigid ring with features that allow the ring to conform to the geometry of a transcatheter valve when implanted. The mitral repair ring is based on existing mitral repair rings and will maintain the shape and relative rigidity needed for ischemic mitral valve repair. At the same time, the design of the new ring will allow for implantation of a transcatheter valve in the mitral position.

In an exemplary example, an annuloplasty ring for mitral valve remodeling and adapted for a valve-in-ring procedure comprises a solid inner core defining a continuous peripheral shape and a suture-permeable tubular interface surrounding the inner core. The inner core is formed of a semi-rigid remodeling material that will resist distortion when subjected to the physiological forces imparted thereon by the mitral valve annulus of an operating human heart. The peripheral shape is generally a D-shape with a substantially straight anterior side diametrically across from a more rounded posterior side with arcuate lateral sides therebetween, and the peripheral shape is oriented around a central axis at an intersection of a minor axis across the midpoints of the anterior and posterior sides and a major axis across the lateral sides. The inner core has radially thick and thin regions along discrete segments around the peripheral shape configured to enable different rates of bending when an internal dilatory force is introduced within the annuloplasty ring and the thick and thin regions are positioned to urge the D-shaped periphery to a more circular shape.

The inner core may have a first radially thick region at least along the posterior side relative to adjacent lateral sides. The first radially thick region is about twice the radial thickness of the lateral sides. The inner core may further have a second radially thick region along the anterior side relative to adjacent lateral sides, wherein the first and second radially thick regions are about twice the radial thickness of the lateral sides. In one example, the second radially thick region has a plurality of spaced radially-outwardly opening notches formed therein.

Another example of an annuloplasty ring for mitral valve remodeling adapted for a valve-in-ring procedure comprises a solid inner core defining a continuous peripheral shape and a suture-permeable tubular interface surrounding the inner core. The inner core is formed of a semi-rigid remodeling material that will resist distortion when subjected to the physiological forces imparted thereon by the mitral valve annulus of an operating human heart. The peripheral shape is generally a D-shape with a substantially straight anterior side diametrically across from a more rounded posterior side with arcuate lateral sides therebetween, and the peripheral shape is oriented around a central axis at an intersection of a minor axis across the midpoints of the anterior and posterior sides and a major axis across the lateral sides. The inner core further has radial slits opening to both inner and outer edges around the peripheral shape configured to enable different rates of bending along discrete segments of the inner core when an internal dilatory force is introduced within the annuloplasty ring, and the radial slits are positioned to urge the D-shaped periphery to a more circular shape.

There are preferably a first plurality of the radial slits opening outwardly and spaced along the anterior side, and a second plurality of the radial slits opening outwardly and spaced along the posterior side. There also may be plurality of the radial slits opening inwardly and spaced along the lateral sides. Each of the radial slits may be formed by a substantially straight channel extending radially from the respective edge of the inner core to an enlarged closed relief area, and each of the closed relief areas may be circular.

The inner core of the rings disclosed herein are preferably formed with a solid cross-section and made of a semi-rigid metal, such as nitinol, cobalt-chromium, stainless steel or a titanium alloy. The peripheral shape may be asymmetric across the minor axis, and the peripheral shape may be three-dimensional and bow down on one side of the minor axis from the anterior side.

The annuloplasty ring may further including at least one expansion joint around the peripheral shape along the inner core which enables a circumference of the inner core to expand when subjected to the internal dilatory force. A singular expansion joint may be located on an anterior side of the D-shaped inner core, or two expansion joints may be located on opposite sides of the D-shaped inner core generally along a major axis thereof.

A combination of an annuloplasty ring for mitral valve remodeling adapted for a valve-in-ring procedure and an expandable prosthetic heart valve is also disclosed. The annuloplasty ring may be configured in any of the aforementioned ways. The combination features an expandable prosthetic heart valve having an expandable support frame made to plastically expand with a generally circular cross-section when subjected to an outward dilatory force. The support frame is covered by a fabric cover surrounded by an attached sealing sleeve formed of a compressible or inelastic material. The heart valve has a contracted shape for advancement within the annuloplasty ring and an expanded shape sized to contact and expand the annuloplasty ring and convert the peripheral shape to a more circular shape, wherein the sealing sleeve expands into gaps between the heart valve and annuloplasty ring and is adapted to form radially thicker and thinner regions around a circumference of the heart valve.

In the aforementioned combination, the inner core may have radially thick and thin regions along discrete segments around the peripheral shape configured to enable different rates of bending when the internal dilatory force is introduced within the annuloplasty ring. Alternatively, the inner core has radial slits opening to both inner and outer edges around the peripheral shape configured to enable different rates of bending along discrete segments of the inner core when the internal dilatory force is introduced within the annuloplasty ring. The annuloplasty ring may further include at least one expansion joint around the peripheral shape of the annuloplasty ring which enables a circumference of the inner core to expand when subjected to the internal dilatory force.

The sealing sleeve desirably attaches to the fabric cover along one circumferential line so as to decouple axial foreshortening of the expandable support frame from the sealing sleeve. The sealing sleeve may comprise an annular bladder having inner and outer walls holding a fluid therein, and the annular bladder may be made of polyethylene terephthalate and the fluid is silicone. Or the sealing sleeve may comprise a solid material which is plastically deformable such as a hydrogel.

A further understanding of the nature and advantages of the disclosure will become apparent by reference to the remaining portions of the specification and drawings.

### BRIEF DESCRIPTION OF THE DRAWINGS

Features and advantages of the present disclosure will become appreciated as the same become better understood with reference to the specification, claims, and appended drawings wherein:
Figure 1 is a perspective view of an exemplary mitral annuloplasty ring having a variable cross-sectional shape that expands subject to an internal dilatory force to a substantially circular peripheral shape;
Figures 2A-2C are plan and elevational views of the mitral annuloplasty ring of Figure 1, and Figures 2D-2F are radial sectional views taken around the periphery thereof;
Figure 3A is a schematic plan view of the mitral annuloplasty ring of Figure 1 undergoing expansion from an internal dilatory force, and Figure 3B is a plan view of the expanded mitral annuloplasty ring showing imposition of an expanded prosthetic heart valve therein;
Figure 4A is a perspective view and Figure 4B a plan view of an exemplary mitral annuloplasty ring having a variable cross-sectional shape that expands subject to an internal dilatory force to a substantially circular peripheral shape;
Figure 5A is a perspective view and Figure 5B a plan view of an exemplary mitral annuloplasty ring having a variable cross-sectional shape that expands subject to an internal dilatory force to a substantially circular peripheral shape;
Figure 6 is a perspective view of another exemplary mitral annuloplasty ring core having a plurality of expansion slits therein that expands subject to an internal dilatory force to a substantially circular peripheral shape;
Figures 7A-7C are plan and elevational views of the mitral annuloplasty ring core of Figure 6;
Figure 8A is a schematic plan view of the mitral annuloplasty ring core of Figure 6 undergoing expansion from an internal dilatory force, and Figure 8B is a plan view of the expanded mitral annuloplasty ring core showing imposition of an expanded prosthetic heart valve therein;
Figure 9A is an elevational view of a prosthetic heart valve having an external sealing sleeve crimped onto an expansion balloon, and Figure 9B is a radial sectional view through the assembly showing the sealing sleeve;
Figure 10 is an elevational view of the prosthetic heart valve with external sealing sleeve after expansion by the balloon;
Figure 11 is a plan view of the expanded balloon and prosthetic heart valve of Figure 10 imposed within the expanded mitral annuloplasty ring core of Figure 3A and showing migration of the sealing sleeve into the gaps between the two;
Figure 12 is a plan view of the expanded balloon and prosthetic heart valve of Figure 10 imposed within the expanded mitral annuloplasty ring core of Figure 8A and showing migration of the sealing sleeve into the gaps between the two;
Figure 13 is a perspective view of an alternative mitral annuloplasty ring core having an expansion joint located on an anterior side thereof, and Figure 14 is a reverse perspective view showing an expansion member exploded from within an expansion slot;
Figures 15A is a schematic plan view of the mitral annuloplasty ring core of Figure 13 undergoing expansion from an internal dilatory force, and Figure 15B is a plan view of the expanded mitral annuloplasty ring core showing imposition of an expanded prosthetic heart valve therein;
Figure 16A is a plan view of a still further mitral annuloplasty ring core having two expansion joints opposite each other on lateral sides, and Figure 16B is a plan view of the expanded mitral annuloplasty ring core showing imposition of an expanded prosthetic heart valve therein;
Figure 17 is a plan view of the expanded balloon and prosthetic heart valve of Figure 10 imposed within the expanded mitral annuloplasty ring core of Figure 13 and showing migration of the sealing sleeve into the gaps between the two; and
Figure 18 is a plan view of the expanded balloon and prosthetic heart valve of Figure 10 imposed within the expanded mitral annuloplasty ring core of Figure 16A and showing migration of the sealing sleeve into the gaps between the two.

### DETAILED DESCRIPTION

The present application discloses a mitral annuloplasty ring that . The term "ring" is used here since the implant is a closed or continuous ring, although in some contexts such implants are also termed "bands." A preferred plan view shape of the disclosed rings is kidney or D-shaped so as to conform to the peripheral shape of the usual mitral annulus.

The right ventricle RV and left ventricle LV are separated from the right atrium RA and left atrium LA, respectively, by the tricuspid valve TV and mitral valve MV; e.g., the atrioventricular valves. Though correction of the mitral annulus is the primary focus of the present application, it should be understood that certain characteristics of the annuloplasty bands described herein may equally be used to treat the tricuspid valve TV, and thus the claims should not be constrained to the mitral band unless expressly limited.

The term "axis" in reference to the illustrated annuloplasty bands, and other non-circular or non-planar bands, refers to a line generally through the centroid of the band or ring periphery when viewed in plan view. "Axial" or the direction of the "axis" can also be viewed as being parallel to the average direction of blood flow within the valve orifice and thus within the band when implanted therein. Stated another way, an implanted mitral band or ring orients about a central flow axis aligned along an average direction of blood flow through the mitral annulus from the left atrium to the left ventricle.

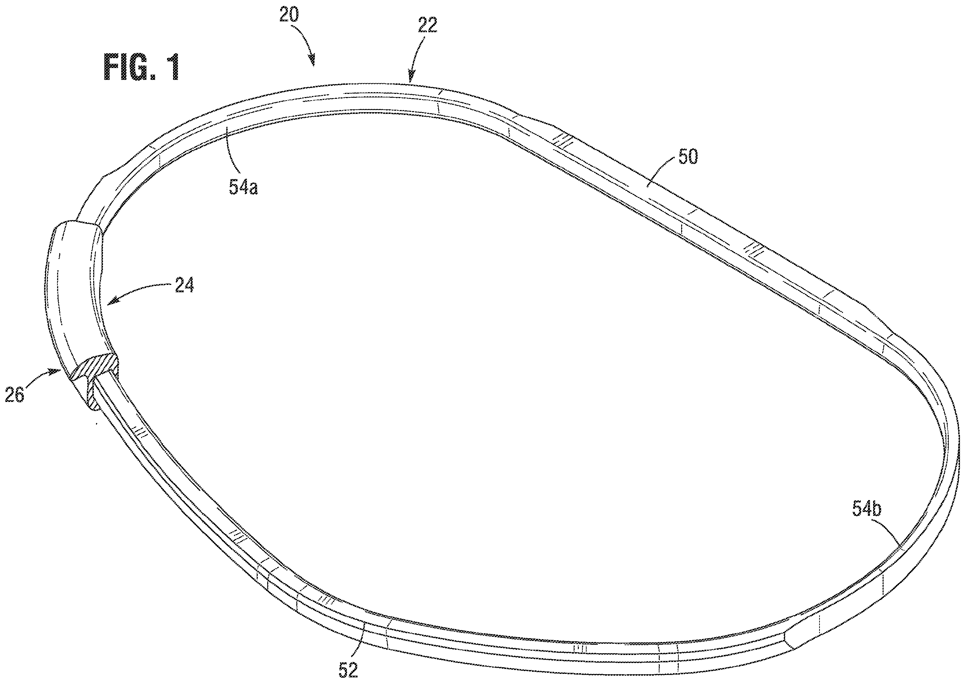

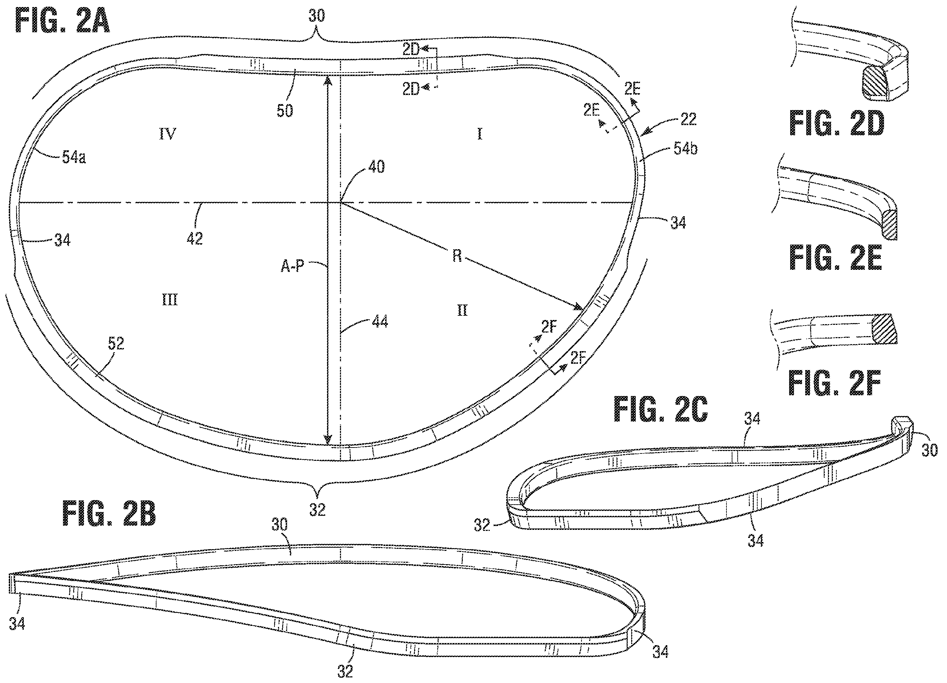

Figure 1 is a perspective view of an exemplary mitral annuloplasty ring 20 having a variable cross-sectional shape, and Figures 2A-2C are plan and elevational views thereof. The annuloplasty ring 20 is configured to expand subject to an internal dilatory force to a substantially circular peripheral shape.

In a preferred example, the ring construction includes a relatively rigid inner core 22 formed in a continuous ring and surrounded by a suture-permeable tubular interface 24, only a segment of which is shown in cutaway. In the illustrated example, the inner core 22 is a solid cross-section, with a greater axial dimension than radial dimension. The core material is a "semi-rigid" metal meaning it will flex but is stiff enough to resist permanent distortion when subjected to the physiological forces imparted thereon by the mitral valve annulus of an operating human heart. In other words, the annulus conforms to the ring instead of the other way around. Preferably, the material of the inner core 22 is an elastic-plastic metal such as nitinol, cobalt-chromium, stainless steel or titanium alloy. This applies to all of the core examples disclosed herein, whether or not there are expansion joints or not.

The tubular interface 24 may include an elastomeric sleeve closely surrounding the entire core and a fabric outer cover (not shown), for example, a polyethylene terephthalate (PET) fabric cover. In the preferred example, the elastomeric sleeve, which may be silicone rubber, is molded to have a radially outwardly-extending flange 26 to facilitate suturing of the ring 20 to the mitral annulus. The ring 20 may be secured with sutures, staples, or other such devices to an inside ledge of the mitral annulus. In a typical procedure, an array of sutures is anchored through the annulus and then threaded through corresponding locations around the interface on the outside of the ring 20, and then the ring is parachuted down the suture array to be seated at the annulus before tying off the sutures.

With reference now to Figures 2A-2C, the D-shaped ring core 22 defines a substantially straight anterior side 30 opposite a more curved posterior side 32, with arcuate lateral sides 34 therebetween. The ring core 22 forms the primary structural component of the annuloplasty ring 20, and thus defines the rounded D-shape of the ring as mentioned. As with many mitral annuloplasty rings, the inner core 22 is arranged around a central flow axis 40 at the intersection of a major axis 42 and a minor axis 44. The dimension across the inner core 22 along the minor axis 44 is denoted the A-P dimension, for anterior-posterior.

The annuloplasty ring 20 may be three-dimensional with an upward bow in the anterior side 30 and a downward bow on the posterior side 32, as seen in Figures 2B and 2C. The ring 20 as seen from above in Figure 2A may be segregated into four quadrants (I-II-III-IV) as defined by the major and minor axes 42, 44 starting from the upper right and going clockwise. The inner core 22 begins to dip downward at a point in the first quadrant and climbs back up in the third quadrant. (It should be noted that a majority of the ring 20 is below the upper middle of the anterior side 30, and the asymmetric downward bow in quadrants I and II is relative to the mirror-image quadrants III and IV on the opposite side.) Additionally, the "radial" distance R between the inner core 22 and the central flow axis 40 is reduced in the second quadrant and a portion of the first quadrant relative to the opposite side across the minor axis 44. This asymmetry across the minor axis 44 in plan and elevational views is believed to better correct for certain pathologies such as ischemic mitral regurgitation (IMR). The shape of the ring 20 is similar to that of the Carpentier-McCarthy-Adams IMR ETlogix^{®} annuloplasty ring available from Edwards Lifesciences of Irvine, CA.

In contrast to earlier mitral annuloplasty rings, the ring core 22 includes thin and thick regions that help determine a final expanded shape upon application of an internal dilatory force. More particularly, the thin and thick regions along discrete segments around the peripheral shape of the ring core 22 cause different rates of bending when an internal dilatory force is introduced within the annuloplasty ring 20 to urge the D-shaped periphery to a more circular shape. The term "internal dilatory force" means an outward expansion force from within the ring 20. Such an expansion force may be applied by a balloon by itself, or a by a balloon expanding a prosthetic heart valve outward into contact with the mitral annulus and pre-implanted annuloplasty ring in a valve-in-ring procedure. It should be understood that the magnitude of an internal dilatory force is significantly greater than physiological forces imparted to the ring by the natural cyclic systolic-diastolic motion of the heart. For instance, the magnitude of an internal dilatory force required to expand a ring core as disclosed herein is greater than 1 atm (760 mm Hg).

As explained above, the inner core 22 comprises a continuous elastic-plastic metal such as nitinol, cobalt-chromium stainless steel or titanium alloy. Typically, such structures present a relatively rigid peripheral shape which is difficult to expand. Moreover, the plan view shape of the ring core 22, as seen in Figure 2A is a rounded D-shape so as to mimic the shape of the native mitral annulus. However, expandable prosthetic heart valves used in valve-in-ring procedures have substantially cylindrical exterior profiles which do not match the rounded D-shape. As a result, gaps are formed around the expanded heart valve which may lead to regurgitation.

With reference back to Figure 1, and also with reference to Figures 2A-2F, the inner core 22 includes a first radially thick region 50 located on the anterior side 30, a second radially thick region 52 located on the posterior side 32, and radially thin regions 54a, 54b located at the arcuate lateral sides 34. The sectional shapes and relative sizes of these regions 50, 52, 54 are shown in Figures 2D-2F. It should be noted that although only one of the thin regions 54b is shown in section, the other thin region 54a may be identical. The inner core 22 has a solid generally rectangular cross-section, in that there are generally four surfaces; namely, top, bottom, inner and outer faces. In the thick regions 50, 52 shown in Figures 2D and 2F, at the anterior and posterior sides 30, 32, respectively, the outer face may be angled inward toward the upper corner with the top and bottom faces being generally parallel and at roughly 90° angles to the inner face. The thin region 54a cross-section of Figure 2E at the lateral sides 34 is thinner radially than the thick regions 50, 52, but the outer face is roughly parallel to the inner face. In all cases the corners between the inner face and the top and bottom faces are rounded to help avoid any abrasion of the valve leaflets moving within the ring. In one example, the axial dimension of the ring core 22 is substantially constant around the periphery, though the radial thickness at the lateral sides 34 is about one half of the radial thickness at the anterior and posterior sides 30, 32. In one example, the boundary between the thick and thin regions is tapered rather than being abrupt.

In absolute terms, a typical ring radial thickness is about .05 inches (1.27 mm). The thin regions may be reduced by half, to about .025 inches (0.64 mm). Although a 50% reduction from the thick to the thin regions is considered effective, the thickness reduction may be more or less than 50%, primarily depending on how much deformation is desired. Therefore, a ratio of the radial dimension between the thin and thick regions is preferably about 50%, but may be down to 25%, and up to 80%. Furthermore, the ring cores shown have discrete spans of either thick or thin regions in a binary pattern, whereas more spans of intermediate thicknesses may be utilized, such as by providing a constantly varying periphery with no abrupt changes whatsoever. One of skill in the art will understand that the ring cores may be customized to perform in innumerable ways when stressed by altering the thickness around the periphery.

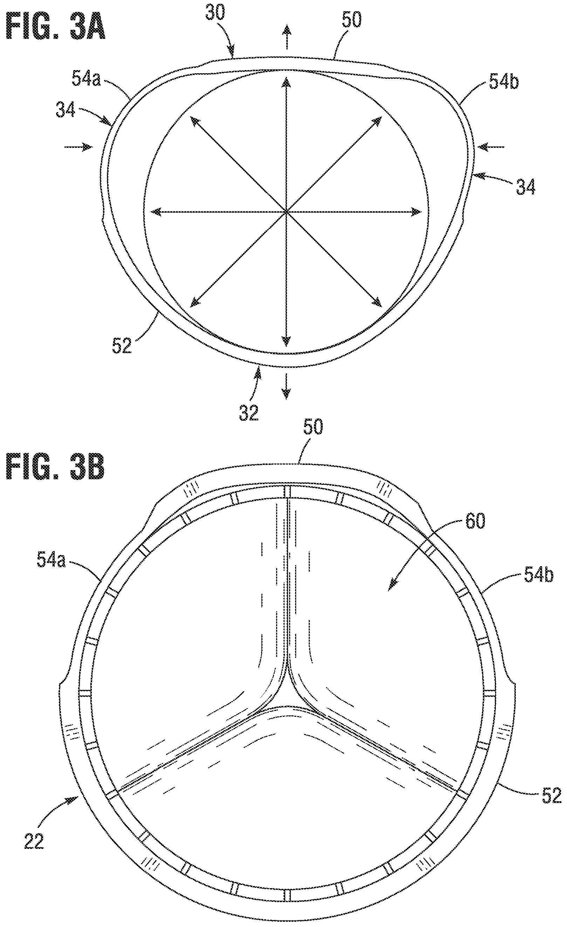

Placement of the thick and thin regions 50, 52, 54 around the periphery of the inner core 22 is designed to encourage the core to expand into a circular shape. For instance, Figure 3A is a schematic plan view of the mitral annuloplasty ring 20 undergoing expansion from an internal generally axi-symmetric dilatory force, represented by a circle with expansion arrows to simulate a balloon or balloon and expanding prosthetic heart valve. Because the dimension along the minor axis 44 of the ring 20 is less than the dimension across the major axis 42, the expanding structure initially contacts the anterior and posterior sides 30, 32, thus forcing them apart. Because these sides 30, 32 incorporate the thick regions 50, 52, they do not deform as much as the thin regions 54a, 54b. Moreover, due to the relatively greater flexibility of the thin regions 54a, 54b, the lateral sides 34 tend to straighten out and move together, as seen. Eventually, the entire structure of the core 22 becomes more circular. In other words, the different thickness regions 50, 52, 54 around the periphery of the ring core 22 experience different rates of bending to urge the D-shaped periphery to a more circular shape.

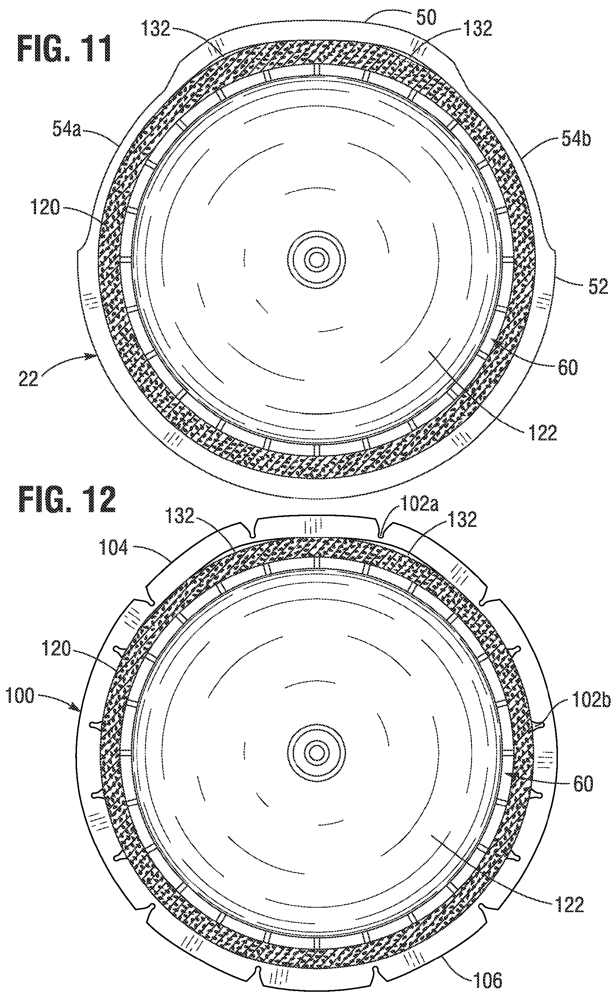

Figure 3B is a plan view of the expanded mitral annuloplasty ring 20 (with the outer suture-permeable tubular interface 24 removed for clarity) showing imposition of an expanded prosthetic heart valve 60 therein. The heart valve 60 may be self-expanding or balloon-expanding. If the former, a dilatory balloon may be first used to expand the annulus and annuloplasty ring 20, and then the heart valve 60 is advanced and permitted to expand within the ring. If the latter, the dilatory balloon expands the heart valve 60 directly outward into contact with the annulus and the annuloplasty ring 20. Although the illustration is a simulation, and the ultimate peripheral shape of the annuloplasty ring 20 may not be entirely circular, those of skill in the art will understand that the presence of the more flexible thin regions 54a, 54b enables the core 22 to more faithfully expand toward a circular shape. This minimizes gaps between the annuloplasty ring 20 and the heart valve 60 therewithin, thus minimizing any potential regurgitation therebetween. Moreover, the exterior of the heart valve 60 may be provided with sealing structures such as fabric skirts and the like to further reduce any regurgitation, as described below with reference to Figure 11.

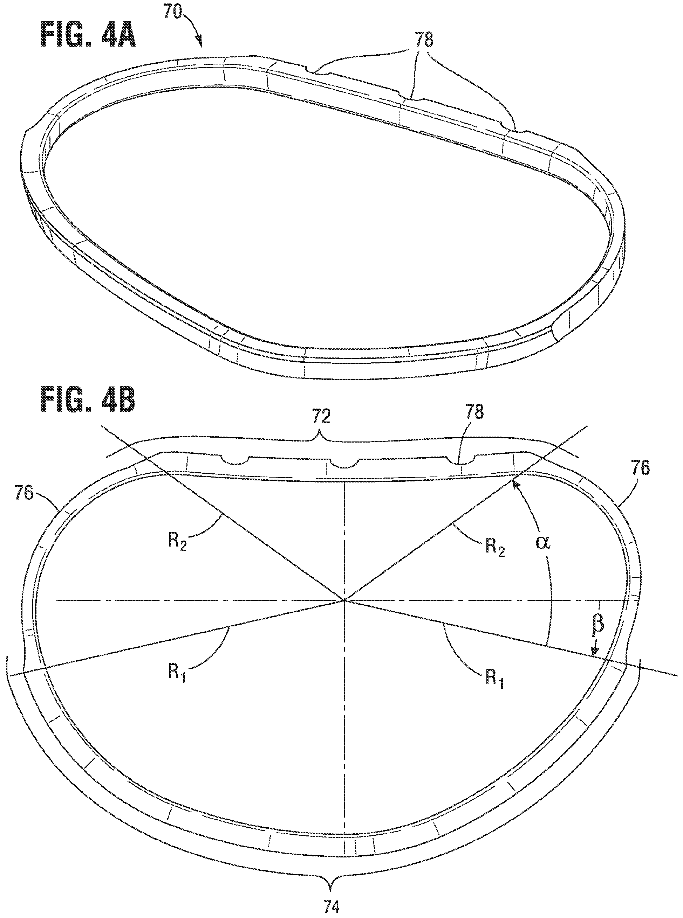

Figure 4A is a perspective view and Figure 4B a plan view of an exemplary mitral annuloplasty ring core 70 similar to the ring core 22 shown in Figures 1-2 with a variable cross-sectional shape that expands subject to an internal dilatory force to a substantially circular peripheral shape. The annuloplasty ring comprises the inner core 70 as shown and typically has a suture-permeable tubular interface surrounding the core for anchoring the ring at the annulus which is not shown for clarity.

As with the earlier core 22, the ring core 70 has an asymmetric D-shape in plan view with a straighter anterior side 72 opposite a more rounded posterior side 74, and lateral sides 76 therebetween. A radial thickness of the ring core 70 is greater along both the anterior side 72 and the posterior side 74 than at the lateral sides 76. In particular, a first radially thick region extends along the posterior side 74 relative to adjacent lateral sides 76, and a second radially thick region extends along the anterior side 72 also relative to adjacent lateral sides. The relative thicknesses may be as described above with respect to the core 22. To help the thickened anterior side 72 more easily conform to an expanding cylindrical dilatory force, a series of outwardly opening notches 78 are provided. In particular, three generally semi-circular notches 78 are evenly spaced along the anterior side 72. These notches reduce the bending strength of the anterior side 72, thus facilitating its transformation from the substantially straight configuration shown to a more rounded arcuate shape after expansion.

Figure 4B shows an exemplary span for the first and second radially thick regions. As described above, the D-shaped mitral ring core 70 is arranged about perpendicular major and minor axes that intersect at a central axis. Although not circular, the ring core 70 is continuous and thus extends 360° around the central axis, and various angular spans may be described using such nomenclature. The first radially thick region extending along the posterior side 74 spans between the border radial lines R₁, and the second radially thick region extending along the anterior side 72 spans between the border radial lines R₂. The radial lines R₁ and R₂ on each lateral side are separated by a first angle α, and a second angle β is formed between the radial lines R₁ and the major axis. The first radially thick region extending along the posterior side 74 spans a greater angle than does the second radially thick region extending along the anterior side 72, and is preferably between 1.5-2 times as long.

The total span of the first and the second radially thick regions equals 360° - 2(α). For the purpose of an illustrative example, the angle α = 45°, and so the total span of the first and the second radially thick regions equals 270° (360° - 90°). In one example, the angle β = 10°, and so the span of the first radially thick region equals 160° (180° - 20°). Consequently, the span of the second radially thick region equals 90° (270° - 160°), and the span of the second radially thick region is about 1.8 times the span of the second radially thick region. Many variations on these spans are contemplated, and the range of angles are desirably 40° > α > 60° and 5° > β > 20°. It should be understood that the spans and exemplary angular ranges described with respect to Figure 4B apply to all of the ring cores disclosed herein having radially thick and thin regions.

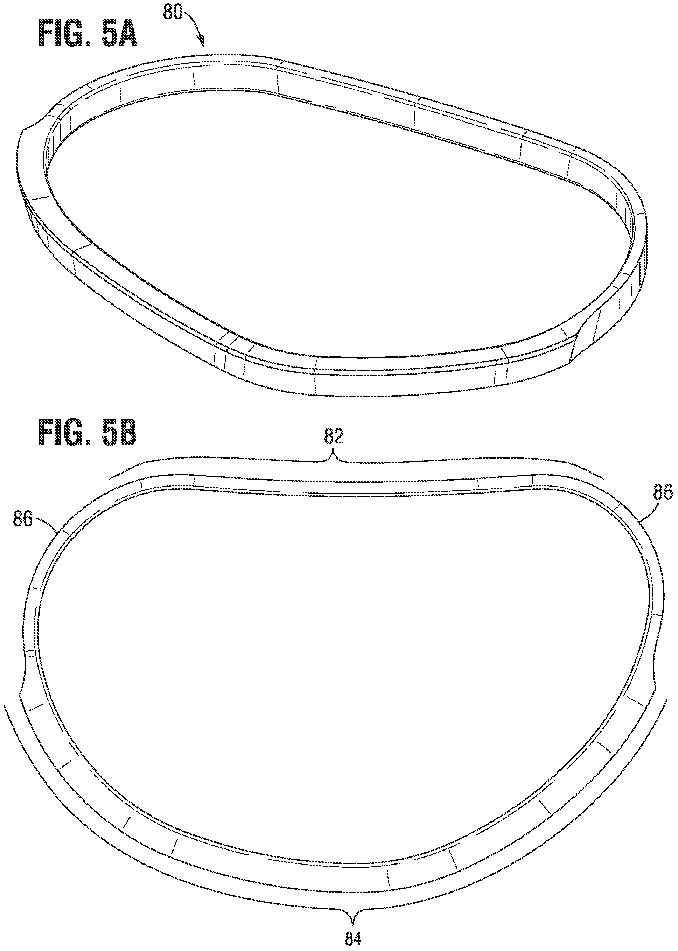

Figure 5A is a perspective view and Figure 5B a plan view of a further exemplary mitral annuloplasty ring core 80 having a variable cross-sectional shape that again is configured to expand subject to an internal dilatory force to a substantially circular peripheral shape. Like the ring core 70, the ring core 80 has an asymmetric D-shape in plan view with a straighter anterior side 82 opposite a more rounded posterior side 84, and lateral sides 86 therebetween. A radial thickness of the ring core 80 is greater along just the posterior side 84 than along the anterior side 82 and the lateral sides 86. This helps ensure that the anterior side 82 easily conforms to the cylindrical expansion force imparted by a balloon, or balloon acting on a prosthetic heart valve in a valve-in-ring procedure. Once again, the relative thicknesses of the sections around the ring core 80 may be the same as described above with respect to the ring core 22.

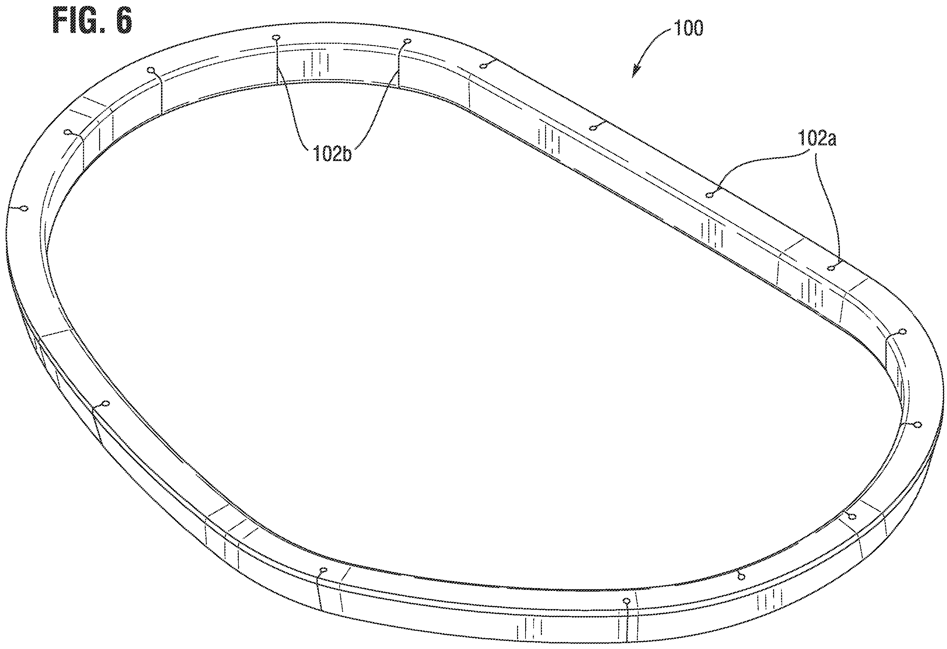

Figure 6 is a perspective view of a different exemplary mitral annuloplasty ring core 100 having a plurality of expansion slits 102a, 102b therein. The slits 102a, 102b enable the ring core 100 to expand subject to an internal dilatory force to a substantially circular peripheral shape, as will be seen. Although not shown, a suture-permeable tubular interface typically surrounds the ring core 100 for passage of sutures anchoring the ring to the annulus.

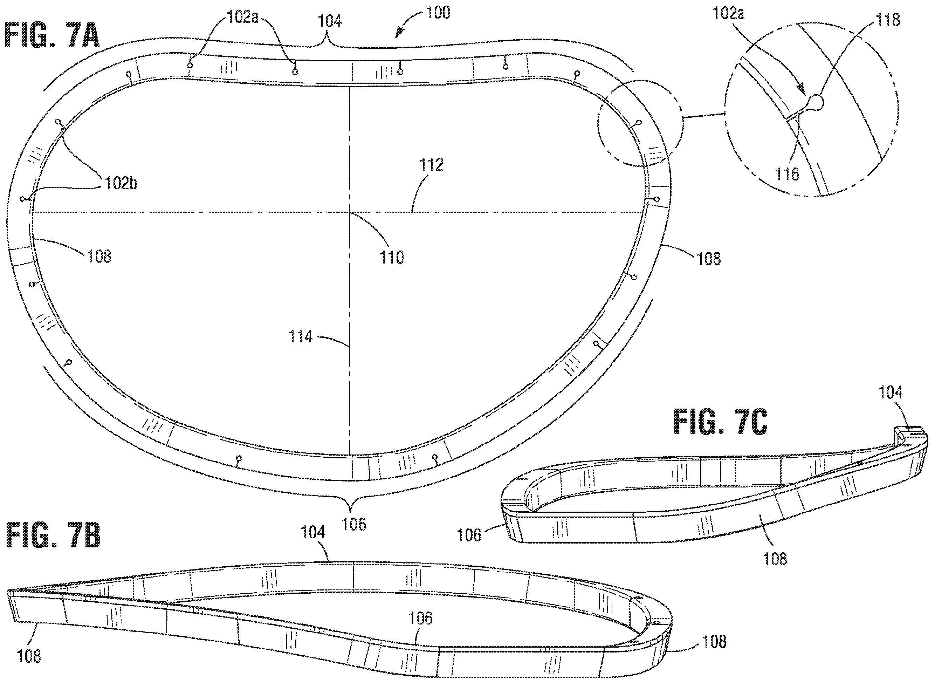

Figures 5A-5C are plan and elevational views of the mitral annuloplasty ring core 100 illustrating the aforementioned asymmetric D-shaped periphery. As before, a generally straight anterior side 104 lies opposite a curved posterior side 106, with lateral sides 108 therebetween. Once again, the ring core 100 defines a central flow axis 110 at the intersection of a major axis 112 and a minor axis 114. The anterior side 104 is at the highest axial elevation, with the posterior side 106 being lower, and quadrant II (see Figure 2A) being at the lowest elevation. In plan view as seen in Figure 7A, the ring core 100 around a portion of quadrants I and II is pulled in somewhat relative to the opposite quadrants diametrically across the minor axis 114.

Now with reference to Figure 7A, the ring core 100 is seen to have exterior slits 102a spaced around the outside periphery and interior slits 102b spaced around the inside periphery. One of the interior slits 102b is seen in large to the right and comprises a substantially straight channel 116 extending radially from the inside periphery to an enlarged closed relief area 118. In the illustrated example, the closed relief area 118 is substantially circular, but may be formed in other shapes. In the illustrated example, there are a series of exterior slits 102a spaced along both the anterior side 104 and the posterior side 106, with interior slits 102b spaced around both lateral sides 108. More specifically, there are four exterior slits 102a spaced along both the anterior and posterior sides 104, 106, and four interior slits 102b spaced on both lateral sides 108. The number and spacing of the interior and exterior slits 102a, 102b may be modified from the pattern shown, but the placement of the interior and exterior slits relative to the periphery of the ring will generally remain the same so as to encourage a more circular expanded shape. Namely, the radial slits 102a, 102b opening to both inner and outer edges enable different rates of bending along certain segments of the ring core 100 that results in an expanded core being more circular than a core without the slits.

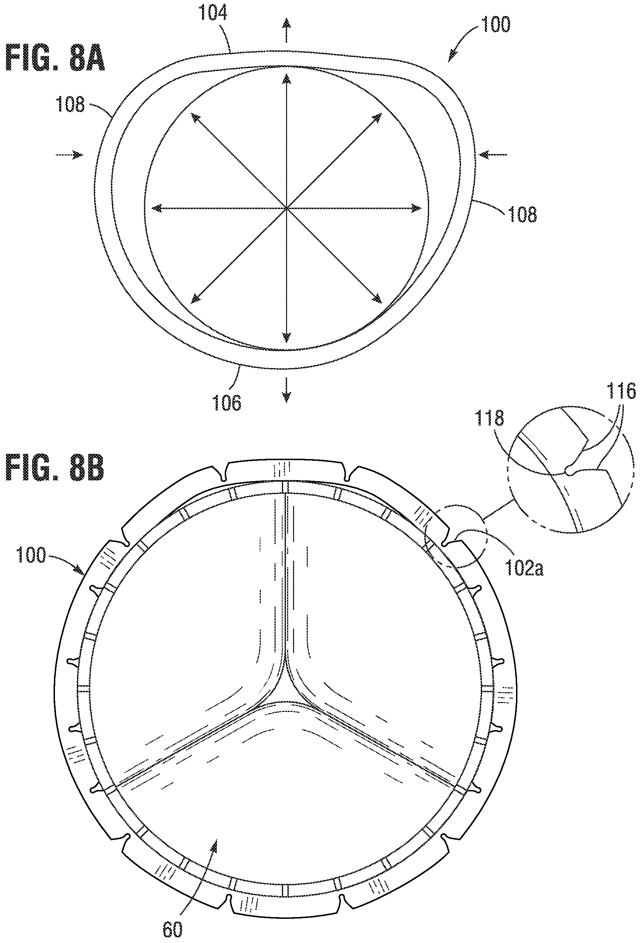

Figure 8A is a schematic plan view of the mitral annuloplasty ring core 100 undergoing expansion from an internal dilatory force, and Figure 8B is a plan view of the expanded mitral annuloplasty ring core showing imposition of an expanded prosthetic heart valve 60 therein. In general, as indicated by the deformation arrows in Figure 8A, the anterior and posterior sides 104, 106 are urged apart, while the lateral sides 108 constrict inward toward each other.

Figure 8B illustrates the effect of the internal dilatory force on one of the external slits 102a. In particular, the sides of the channel 116 spread apart and the enlarged relief area 118 provides a stop to further crack propagation. That is, the material of the ring core 100 is desirably nitinol, cobalt-chromium, stainless steel or titanium, and the rounded relief area 118 helps prevent complete severing of the ring core at that point in contrast to a slit without the relief area which might continue through the material.

Each of the external and internal slits 102a, 102b undergoes the spreading apart, though some may spread more than the others. The depth of the slits 102a, 102b also has an effect on how wide the slits spread at any one point. In one example, each of the slits 102a, 102b extends about half-way radially through the ring core cross-section. In general, because the anterior and posterior sides 104, 106 are forced radially outward, the exterior slits 102a allow the ring core 100 along the sides to constrict inward toward the ultimately circular heart valve 60. In contrast, as the lateral sides 108 contract inward they become somewhat straighter which is accommodated by expansion of the interior slits 102b. Another way to look at it is that the anterior and posterior sides 104, 106 conform more around the curve of the expanding circular dilatory force, while the posterior sides 108 pull inward to reduce the gaps between them and the expanding circular shape. Those of skill in the art will understand that careful placement, spacing and sizing of the exterior and interior slits 102a, 102b can be used to affect the final expanded shape of variously-shaped ring cores so as to be a circular as possible.

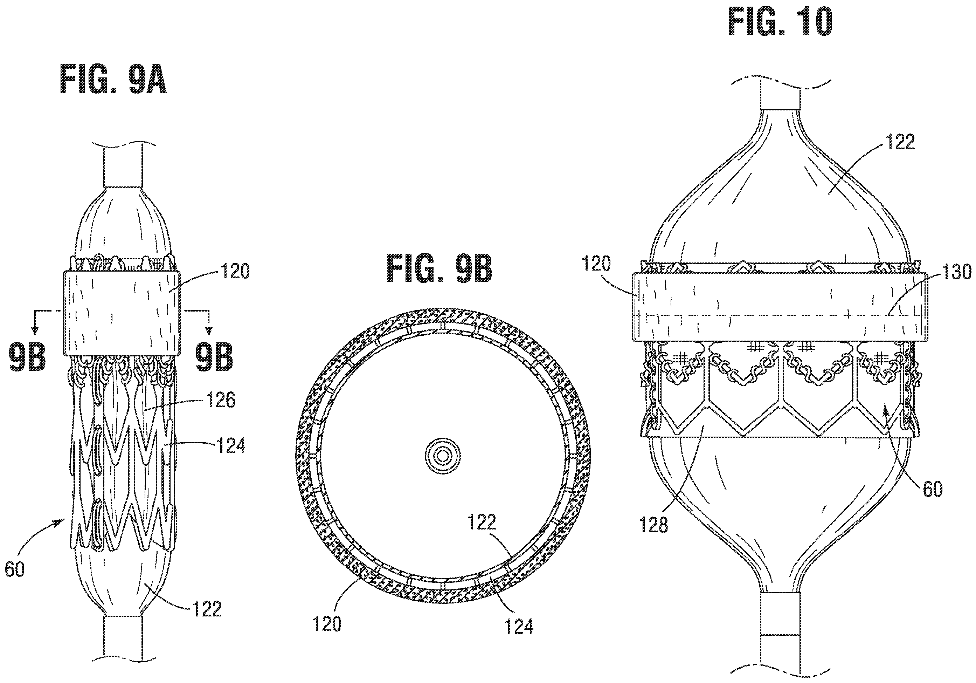

Figure 9A is an elevational view of a prosthetic heart valve 60 having an external sealing sleeve 120 and crimped onto an expansion balloon 122, and Figure 9B is a radial sectional view through the assembly showing the external sealing sleeve. The prosthetic heart valve 60 typically includes a self-or balloon-expandable frame 124 supporting a plurality of flexible bioprosthetic leaflets 126 in a flow orifice therein. In the illustrated example, the frame 124 is balloon-expandable and may be crimped around the expansion balloon 122 for subsequent delivery and expansion at an implantation site.

The external sealing sleeve 120 may comprise a compressible or flowable (inelastic) material, or may be configured as an annular bladder having inner and outer walls holding a fluid therein. Preferably, the external sealing sleeve 120 includes a flexible biocompatible bladder of, for instance, polyethylene terephthalate (PET), with a filling of deformable material such as silicone which may be cured to hold a shape. In another configuration, the sealing sleeve 120 is a solid material (no bladder) which is plastically deformable, having a putty-like quality, such as a hydrogel, that may compress unevenly around the heart valve 60 and end up thicker in some areas than others. Whatever way the sealing sleeve 120 is configured, it is desirably secured around the exterior of the prosthetic heart valve 60. Preferably, the sealing sleeve 120 has an axial dimension sufficient to extend around the part of the prosthetic heart valve 60 containing the inflow end of the bioprosthetic leaflets 126. For instance, the prosthetic heart valve 60 may have a crimped axial length of about 30 mm, while the sealing sleeve 120 has an axial length of about 10 mm. When the prosthetic heart valve 60 expands, both it and the sealing sleeve 124 shorten somewhat in the axial direction, but the proportional axial lengths remain approximately the same.

Figure 10 is an elevational view of the prosthetic heart valve 60 with external sealing sleeve 120 after expansion by the balloon 122. As mentioned, the heart valve 60 and the sealing sleeve 120 undergo some axial foreshortening, though if the sealing sleeve 120 is not attached along its entire length it may not shorten proportionally as much. For instance, a prosthetic heart valve 60 may shorten from 30 mm to 20 mm, while the sealing sleeve 120 may only shorten from 10 mm to 8 mm. Preferably, the expandable frame 124 also has a fabric skirt or layer 128 extending inside, outside or both to further help prevent paravalvular leakage and to which the sealing sleeve 120 may be secured, such as via a single line of stitching 130, or at least stitching concentrated along one circumferential line to decouple the axial foreshortening of the expandable frame 124 from the sealing sleeve 120.

Figure 11 is a plan view of the expanded balloon 122 and prosthetic heart valve 60 of Figure 10 imposed within the expanded mitral annuloplasty ring core 22 of Figure 3A. As mentioned above with reference to Figure 3B, the presence of the more flexible thin regions 54a, 54b enables the core 22 to more faithfully expand toward a circular shape and minimize gaps 132 between the annuloplasty ring 20 and the heart valve 60. To further prevent paravalvular leaking, the sealing sleeve 120 compresses unevenly and migrates or flows into the gaps 132 that may remain between the two structures. That is, the radial thickness of the sealing sleeve 120 will vary around the circumference of the heart valve 60 after expansion due to the compressibility and/or plastic nature of the material. Depending on the size of the gaps 132 and configuration of the sealing sleeve 120 most of not all of the potential leaks will be plugged.

Likewise, Figure 12 is a plan view of the expanded balloon 122 and prosthetic heart valve 60 of Figure 10 imposed within the expanded mitral annuloplasty ring core 100 of Figure 8A. As mentioned above with reference to Figure 8B, the presence of the external slits 102a enable the core 100 to more faithfully expand toward a circular shape and minimize gaps 132 between the annuloplasty ring and the heart valve 60. To further prevent paravalvular leaking, the sealing sleeve 120 compresses unevenly and migrates into the gaps 132 that may remain between the two structures. Again, depending on the size of the gaps 132 and configuration of the sealing sleeve 120 most of not all of the potential leaks will be plugged.

Up to this point the annuloplasty rings configured to expand to substantially circular peripheral shapes have had solid or otherwise contiguous structural cores, albeit with thick/thin regions or strategically placed slits. The cores expand from D-shaped to more circular shapes, but the total circumferential length remains the same. An alternative core construction with expansion segments may also be utilized which expands to a more circular shape while also undergoing an increased circumferential length. The following examples are illustrative. Although not shown, these expandable core constructions may be combined with the thick/thin regions or have internal and/or external slits as described above.

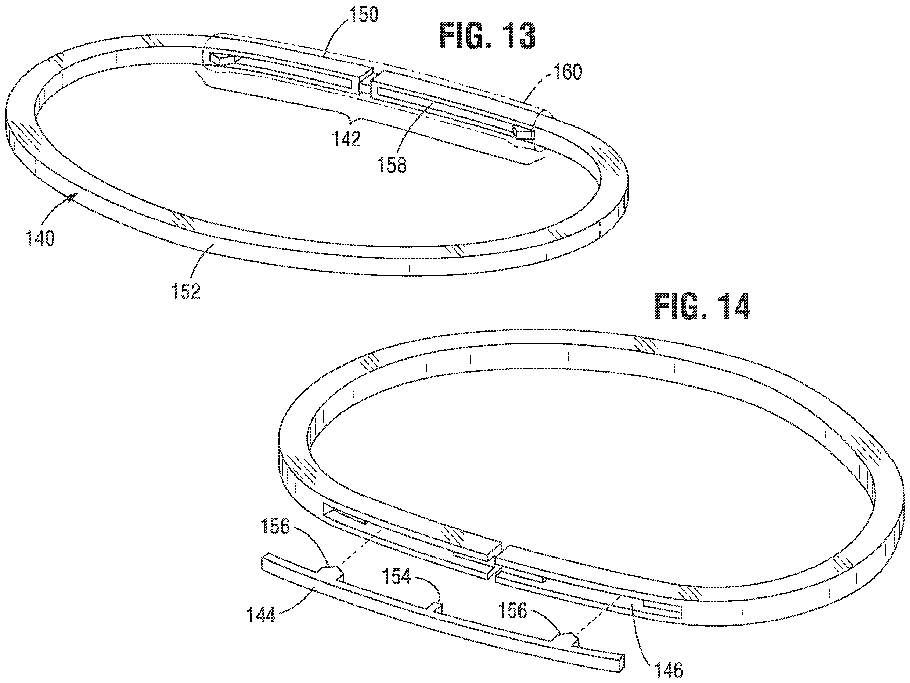

Figure 13 is a perspective view of an alternative mitral annuloplasty ring core 140 having an expansion joint 142 located on an anterior side thereof, and Figure 14 is a reverse perspective view showing an expansion insert 144 exploded from within an expansion slot 146.

The inner core 140 may be shaped substantially the same as described above with a rounded D-shaped periphery defining a straight anterior side 150 opposite a more curved posterior side 152. The anterior side 150 of the inner core 140 has the elongated outwardly-opening circumferential slot 146 which closely receives the expansion insert 144. The expansion insert 144 has a thin arcuate configuration with a central boss 154 extending radially inward as well as a pair of expansion-limiting bosses 156 also extending radially inward from opposite ends. As seen in Figure 13, the anterior side 150 also includes on its inner periphery a pair of spaced apart short slots 158 through which the expansion-limiting bosses 156 project.

The anterior side 150 is bifurcated at its midpoint such that the two abutting ends may be separated due to an internal dilatory expansion force, as described above. In the relaxed or contracted configuration of the inner core 140, the abutting ends of the anterior side 150 prevent the ring circumference from reducing below that of its relaxed state. This allows the ring having the ring core 140 to function in a remodeling capacity, as with the continuous unbroken ring cores of Figs. 1-6. As the free ends of the anterior side 150 separate, the short slots 158 slide outward over the expansion insert 144 until the expansion-limiting bosses 156 reach the other end of the short slots, which prevents further expansion. Each of the expansion-limiting bosses 156 has a triangular inner end which closely engages an inside of a compression sleeve 160 provided around the expansion joints. The compression sleeve 160 helps prevent premature expansion of the inner core 140 prior to application of a dilatory force such as from a balloon or expanding heart valve, and may be formed of a heat-shrinkable polyethylene terephthalate (PET).

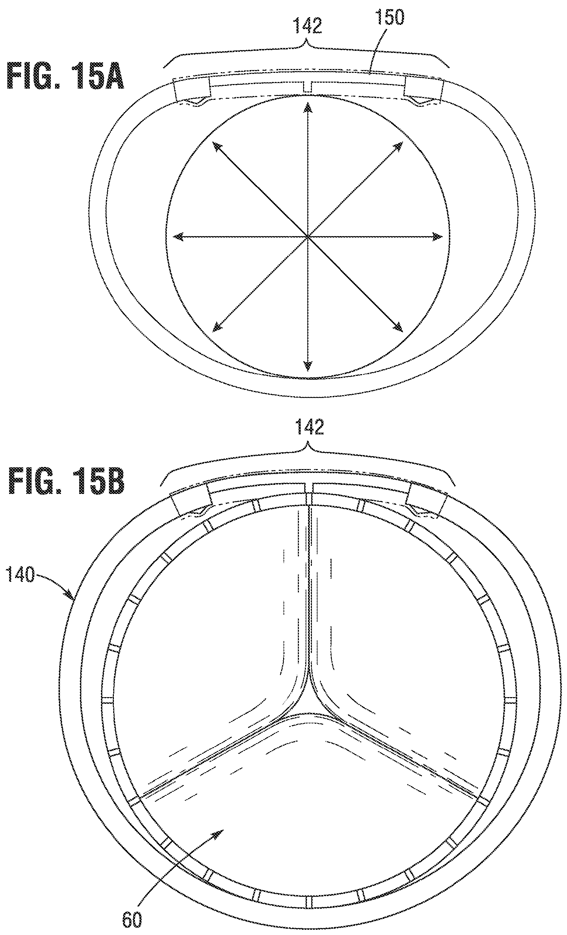

Figure 15A is a schematic plan view of the mitral annuloplasty ring core 140 undergoing expansion from an internal dilatory force, while Figure 15B is a plan view showing imposition of an expanded prosthetic heart valve 60 of generally circular cross-section therein. As with the earlier-described ring cores, the ring core 140 expands along the anterior-posterior dimension (e.g., along the minor axis), while the lateral sides constrict inward. Because the expansion joint 142 is centered in the straight anterior side 150, the overall peripheral shape of the ring core 140 becomes more circular for conforming better around the prosthetic heart valve 60, as seen in Figure 15B. Once again, any gaps that still remain between the outwardly extending prosthetic heart valve 60 are minimized and may be filled with a fabric covering around the heart valve or a conformable cuff, as explained below.

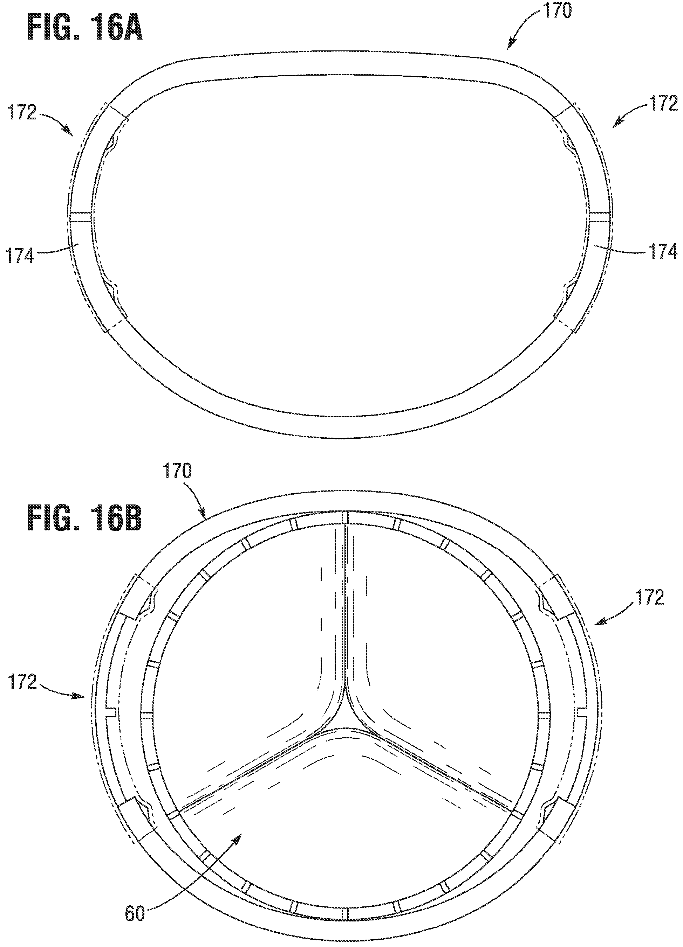

Figure 16A is a plan view of a still further mitral annuloplasty ring core 170 having two expansion joints 172 opposite each other on lateral sides 174, and Figure 16B is a plan view of the expanded mitral annuloplasty ring core showing imposition of an expanded prosthetic heart valve 60 therein. The shape of the ring core 170 will not be described again, but can be presumed to be D-shaped in plan view and three-dimensional as described above. The expansion joints 172 may be similar to the expansion joint 142 described above with respect to Figures 13-15.

As a balloon or balloon within the prosthetic heart valve 60 expands, the expansion joints 172 spread apart which tends to elongate and radially constrict the lateral sides 174. The resulting peripheral shape of the ring core 170 is more circular, as shown. This helps conform the cylindrical heart valve 60 within the ring core 170, thus reducing the leakage therebetween. Again, fabric skirts or cuffs may be provided on the heart valve 60 to further reduce leakage.

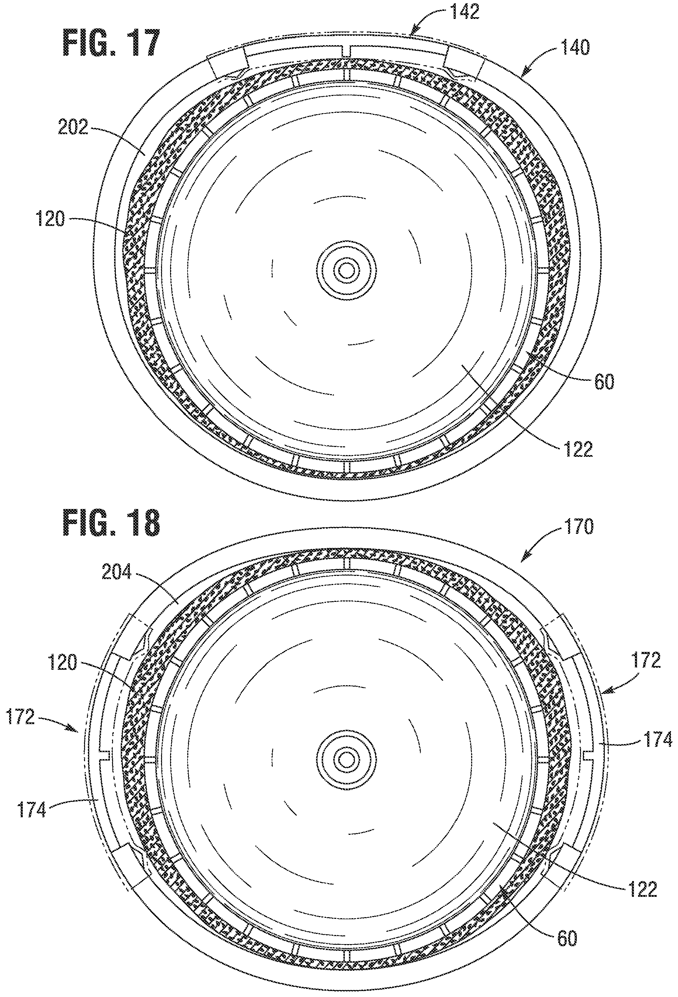

Figure 17 is a plan view of the expanded balloon 122 and prosthetic heart valve 60 imposed within a mitral annuloplasty ring having the ring core 140 of Figure 13. The initially D-shaped ring core 140 has the expansion joint 142 on the anterior side, and thus tends to expand to a more circular shape. Moreover, the sealing sleeve 120 compresses unevenly and migrates into the crescent-shaped gaps 202 that remain between the two structures. Depending on the size of the gaps 202 and configuration of the sealing sleeve 120 most of not all of the gaps may be plugged.

Figure 18 is a plan view of the expanded balloon 122 and prosthetic heart valve 60 imposed within a mitral annuloplasty ring having the ring core 170 of Figure 16A. The initially D-shaped ring core 170 has the expansion joints 172 on both lateral sides 174, and thus tends to expand to a more circular shape. The sealing sleeve 120 compresses unevenly and migrates into the crescent-shaped gaps 204 that remain between the two structures. Again, depending on the size of the gaps 204 and configuration of the sealing sleeve 120, most if not all of the gaps may be plugged.

While the foregoing is a complete description of the preferred examples, various alternatives, and modifications may be used. Moreover, it will be obvious that certain other modifications may be practiced within the scope of the appended claims.

## Claims

1. An annuloplasty ring (20) for mitral valve remodeling adapted for a valve-in-ring procedure, comprising:
an inner core (22; 70; 80; 100) defining a continuous peripheral shape and a suture-permeable tubular interface (24) surrounding the inner core (22; 70; 80; 100), the inner core (22; 70; 80; 100) being formed of a semi-rigid remodeling material that will resist distortion when subjected to the physiological forces imparted thereon by the mitral valve annulus of an operating human heart, the peripheral shape being generally a D-shape with a substantially straight anterior side (30; 72; 82; 104) diametrically across from a more rounded posterior side (32; 74; 84; 106) with arcuate lateral sides (34; 76; 86; 108) therebetween, and the peripheral shape is oriented around a central axis (40; 110) at an intersection of a minor axis (44; 114) across the midpoints of the anterior and posterior sides (32; 74; 84; 106) and a major axis (42; 112) across the lateral sides (34; 76; 86; 108), wherein,
the inner core (22; 70; 80) has radially thick (50, 52) and thin (54a, 54b) regions along discrete segments around the peripheral shape configured to enable different rates of bending when an internal dilatory force is introduced within the annuloplasty ring (20) and the thick and thin regions are positioned to urge the D-shaped peripheral shape to a more circular shape when expanded, or
the inner core (100) has radial slits (102a, 102b) opening to both inner and outer edges around the peripheral shape configured to enable different rates of bending along discrete segments of the inner core (100) when the internal dilatory force is introduced within the annuloplasty ring (20) and the radial slits are positioned to urge the D-shaped peripheral shape to a more circular shape when expanded.

2. The annuloplasty ring (20) of claim 1, wherein the inner core (22; 70; 80) has a first radially thick region (50) at least along the posterior side (32; 74; 84) relative to adjacent lateral sides (34; 76; 86), optionally, wherein the first radially thick region (50) is about twice the radial thickness of the lateral sides (34; 76; 86).

3. The annuloplasty ring (20) of claim 2, wherein the inner core (22; 70; 80) has a second radially thick region (52) along the anterior side (30; 72; 82) relative to adjacent lateral sides (34; 76; 86), optionally, wherein the first and second radially thick regions (50, 52) are about twice the radial thickness of the lateral sides (34; 76; 86).

4. The annuloplasty ring (20) of claim 3, wherein the second radially thick region (52) has a plurality of spaced radially-outwardly opening notches (78) formed therein.

5. The annuloplasty ring (20) of claim 1, wherein there are a first plurality of the radial slits (102a) opening outwardly and spaced along the anterior side (104), optionally, wherein there are a second plurality of the radial slits (102b) opening outwardly and spaced along the posterior side (106).

6. The annuloplasty ring (20) of claim 1 or 5, wherein there are a plurality of the radial slits (102a, 102b) opening inwardly and spaced along the lateral sides (108).

7. The annuloplasty ring (20) of any of claims 1, 5 and 6, wherein each of the radial slits (102a, 102b) is formed by a substantially straight channel (116) extending radially from the respective edge of the inner core (100) to an enlarged closed relief area (118), optionally, wherein each of the closed relief areas (118) are circular.

8. The annuloplasty ring (20) of any of claims 1 to 7, wherein the peripheral shape is asymmetric across the minor axis (44; 114).

9. The annuloplasty ring (20) of claim 8, wherein the peripheral shape is three-dimensional and bows down on one side of the minor axis (44; 114) from the anterior side (30; 72; 82; 104).

10. The annuloplasty ring (20) of any of claims 1 to 9, wherein the inner core (22; 70; 80; 100) is formed with a solid cross-section and made of a semi-rigid metal.

11. The annuloplasty ring (20) of claim 10, wherein the semi-rigid metal is selected from the group consisting of nitinol, cobalt-chromium, stainless steel and a titanium alloy.

12. The annuloplasty ring (20) of any of claims 1 to 9, further including at least one expansion joint (142; 172) around the peripheral shape along the inner core which enables a circumference of the inner core to expand when subjected to the internal dilatory force.

13. A combination of an annuloplasty ring (20) for mitral valve remodelling adapted for a valve-in-ring procedure and an expandable prosthetic heart valve (60), comprising:
an annuloplasty ring (20) of any of claims 1 to 12; and
an expandable prosthetic heart valve (60) having an expandable support frame made to plastically expand with a generally circular cross-section when subjected to an outward dilatory force, the support frame being covered by a fabric cover surrounded by an attached sealing sleeve (120) formed of a compressible or inelastic material, wherein the heart valve (60) has a contracted shape for advancement within the annuloplasty ring (20) and an expanded shape sized to contact and expand the annuloplasty ring (20) and convert the peripheral shape to a more circular shape, wherein the sealing sleeve (120) expands into gaps (132) between the heart valve (60) and annuloplasty ring (20) and is adapted to form radially thicker and thinner regions around a circumference of the heart valve (60), optionally, wherein the sealing sleeve (120) comprises a solid material which is plastically deformable.

14. The combination of claim 13, wherein the sealing sleeve (120) attaches to the fabric cover along one circumferential line so as to decouple axial foreshortening of the expandable support frame from the sealing sleeve (120).

15. The combination of claim 13 or 14, wherein the sealing sleeve (120) comprises an annular bladder having inner and outer walls holding a fluid therein, optionally, wherein the annular bladder is made of polyethylene terephthalate and the fluid is silicone.

## Patentansprüche

1. Anuloplastie-Ring (20) zur Mitralklappen-Remodellierung, eingerichtet für eine Klappe-im-Ring-Prozedur, umfassend:
einen inneren Kern (22; 70; 80; 100), der eine kontinuierliche periphere Form definiert, und eine nahtdurchlässige röhrenförmige Grenzfläche (24), die den inneren Kern (22; 70; 80; 100) umgibt, wobei der innere Kern (22; 70; 80; 100) aus einem halbstarren Remodellierungs-Material gebildet ist, das einer Verformung widersteht, wenn es den physiologischen Kräften ausgesetzt wird, die durch den Mitralklappenanulus eines arbeitenden menschlichen Herzens darauf ausgeübt werden, wobei die periphere Form im Allgemeinen eine D-Form ist mit einer im Wesentlichen geraden anterioren Seite (30; 72; 82; 104) diametral gegenüber einer stärker gerundeten posterioren Seite (32; 74; 84; 106) mit gebogenen lateralen Seiten (34; 76; 86; 108) dazwischen, und wobei die periphere Form um eine zentrale Achse (40; 110) an einem Schnittpunkt einer Nebenachse (44; 114) durch die Mittelpunkte der anterioren und posterioren Seiten (32; 74; 84; 106) und einer Hauptachse (42; 112) durch die lateralen Seiten (34; 76; 86; 108) ausgerichtet ist, wobei,
der innere Kern (22; 70; 80) radial dicke (50, 52) und dünne (54a, 54b) Bereiche entlang diskreter Segmente um die periphere Form herum aufweist, die dazu konfiguriert sind, unterschiedliche Biegungsraten zu ermöglichen, wenn eine innere Dilatationskraft innerhalb des Anuloplastie-Rings (20) eingebracht wird, und die dicken und dünnen Bereiche so positioniert sind, dass sie die D-förmige periphere Form in eine mehr kreisförmige Form zwingen, wenn sie expandiert wird, oder
der innere Kern (100) radiale Schlitze (102a, 102b) aufweist, die sich sowohl zu inneren als auch zu äußeren Rändern um die periphere Form herum öffnen und dazu konfiguriert sind, unterschiedliche Biegungsraten entlang diskreter Segmente des inneren Kerns (100) zu ermöglichen, wenn die innere Dilatationskraft innerhalb des Annuloplastie-Rings (20) eingebracht wird, und die radialen Schlitze so positioniert sind, dass sie die D-förmige periphere Form in eine mehr kreisförmige Form zwingen, wenn sie expandiert wird.

2. Anuloplastie-Ring (20) nach Anspruch 1, wobei der innere Kern (22; 70; 80) zumindest entlang der posterioren Seite (32; 74; 84) relativ zu den benachbarten lateralen Seiten (34; 76; 86) einen ersten radial dicken Bereich (50) aufweist, wobei optional der erste radial dicke Bereich (50) etwa doppelt so dick ist wie die radiale Dicke der lateralen Seiten (34; 76; 86).

3. Anuloplastie-Ring (20) nach Anspruch 2, wobei der innere Kern (22; 70; 80) einen zweiten radial dicken Bereich (52) entlang der anterioren Seite (30; 72; 82) relativ zu den benachbarten lateralen Seiten (34; 76; 86) aufweist, wobei optional der erste und der zweite radial dicke Bereich (50, 52) etwa doppelt so dick sind wie die radiale Dicke der lateralen Seiten (34; 76; 86).

4. Annuloplastie-Ring (20) nach Anspruch 3, wobei der zweite radial dicke Bereich (52) eine Vielzahl von beabstandeten, sich radial nach außen öffnenden Vertiefungen (78) aufweist, die darin ausgebildet sind.

5. Anuloplastie-Ring (20) nach Anspruch 1, wobei eine erste Vielzahl von radialen Schlitzen (102a) vorhanden ist, die sich nach außen öffnen und entlang der anterioren Seite (104) beabstandet angeordnet sind, optional, wobei eine zweite Vielzahl von radialen Schlitzen (102b) vorhanden ist, die sich nach außen öffnen und entlang der posterioren Seite (106) beabstandet angeordnet sind.

6. Anuloplastie-Ring (20) nach Anspruch 1 oder 5, wobei eine Vielzahl von radialen Schlitzen (102a, 102b) vorhanden ist, die sich nach innen öffnen und entlang der lateralen Seiten (108) beabstandet angeordnet sind.

7. Annuloplastie-Ring (20) nach einem der Ansprüche 1, 5 und 6, wobei jeder der radialen Schlitze (102a, 102b) durch einen im Wesentlichen geraden Kanal (116) gebildet wird, der sich radial von dem jeweiligen Rand des inneren Kerns (100) zu einem vergrößerten geschlossenen Entlastungsbereich (118) erstreckt, wobei jeder der geschlossenen Entlastungsbereiche (118) optional kreisförmig ist.

8. Annuloplastie-Ring (20) nach einem der Ansprüche 1 bis 7, wobei die periphere Form über die Nebenachse (44; 114) asymmetrisch ist.

9. Anuloplastie-Ring (20) nach Anspruch 8, wobei die periphere Form dreidimensional ist und sich auf einer Seite der Nebenachse (44; 114) von der anterioren Seite (30; 72; 82; 104) nach unten wölbt.

10. Annuloplastie-Ring (20) nach einem der Ansprüche 1 bis 9, wobei der innere Kern (22; 70; 80; 100) mit einem massiven Querschnitt ausgebildet ist und aus einem halbstarren Metall gefertigt ist.

11. Anuloplastie-Ring (20) nach Anspruch 10, wobei das halbstarre Metall ausgewählt ist aus der Gruppe bestehend aus Nitinol, Kobalt-Chrom, rostfreiem Stahl und einer Titanlegierung.

12. Annuloplastie-Ring (20) nach einem der Ansprüche 1 bis 9, der ferner mindestens eine Expansionsverbindung (142; 172) um die periphere Form entlang des inneren Kerns aufweist, die es ermöglicht, dass ein Umfang des inneren Kerns expandiert, wenn er der inneren Dilatationskraft ausgesetzt ist.

13. Kombination aus einem Anuloplastie-Ring (20) zur Mitralklappen-Remodellierung, der für eine Klappen-im-Ring-Prozedur eingerichtet ist, und einer expandierbaren Herzklappenprothese (60), umfassend:
einen Annuloplastie-Ring (20) nach einem der Ansprüche 1 bis 12; und
eine expandierbare Herzklappenprothese (60), die einen expandierbaren Stützrahmen aufweist, der so gestaltet ist, dass er mit einem allgemein kreisförmigen Querschnitt plastisch expandiert, wenn er einer nach außen gerichteten Dilatationskraft ausgesetzt wird, wobei der Stützrahmen von einer Stoff-Abdeckung bedeckt ist, die von einer angebrachten Dichtungshülle (120) umgeben ist, die aus einem komprimierbaren oder unelastischen Material gebildet ist, wobei die Herzklappe (60) eine kontrahierte Form zum Vorschieben innerhalb des Anuloplastie-Rings (20) und eine expandierte Form aufweist, die so bemessen ist, dass sie den Anuloplastie-Ring (20) berührt und expandiert und die periphere Form in eine mehr kreisförmige Form konvertiert, wobei die Dichtungshülle (120) in Lücken (132) zwischen der Herzklappe (60) und dem Annuloplastie-Ring (20) expandiert und dazu eingerichtet ist, radial dickere und dünnere Bereiche um einen Umfang der Herzklappe (60) zu bilden, wobei optional die Dichtungshülle (120) ein massives Material umfasst, das plastisch verformbar ist.

14. Kombination nach Anspruch 13, bei der die Dichtungshülle (120) entlang einer Umfangslinie an der Stoff-Abdeckung angebracht ist, so dass eine axiale Verkürzung des expandierbaren Stützrahmens von der Dichtungshülle (120) entkoppelt wird.

15. Kombination nach Anspruch 13 oder 14, wobei die Dichtungshülle (120) eine ringförmige Blase mit inneren und äußeren Wänden umfasst, die darin ein Fluid hält, wobei optional die ringförmige Blase aus Polyethylenterephthalat gefertigt ist und das Fluid Silikon ist.

## Revendications

1. Anneau d'annuloplastie (20) pour le remodelage de la valvule mitrale, conçu pour une procédure "valve-in-ring", comprenant :
un noyau interne (22 ; 70 ; 80 ; 100) définissant une forme périphérique continue et une interface tubulaire (24) perméable à la suture entourant le noyau interne (22 ; 70 ; 80 ; 100), le noyau interne (22 ; 70 ; 80 ; 100) étant formé d'un matériau de remodelage semi-rigide qui résiste à la distorsion lorsqu'il est soumis aux forces physiologiques qui lui sont communiquées par l'anneau de valvule mitrale d'un cœur humain en fonctionnement, la forme périphérique étant généralement une forme de D pourvue d'un côté antérieur (30 ; 72 ; 82 ; 104) sensiblement droit diamétralement à travers un côté postérieur (32 ; 74 ; 84 ; 106) plus arrondi et des côtés latéraux arqués (34 ; 76 ; 86 ; 108) entre ceux-ci et la forme périphérique est orientée autour d'un axe central (40 ; 110) au niveau d'une intersection d'un petit axe (44 ; 114) à travers les points médians des côtés antérieur et postérieur (32 ; 74 ; 84 ; 106) et d'un axe principal (42 ; 112) à travers les côtés latéraux (34 ; 76 ; 86 ; 108),
le noyau interne (22 ; 70 ; 80) présentant des régions radialement épaisses (50, 52) et minces (54a, 54b) le long de segments discrets autour de la forme périphérique, conçues pour permettre différents taux de courbure lorsqu'une force de dilatation interne est introduite à l'intérieur de l'anneau d'annuloplastie (20) et les régions épaisses et minces étant positionnées afin de pousser la forme périphérique en forme de D vers une forme plus circulaire lors de la dilatation ou
le noyau interne (100) présentant des fentes radiales (102a, 102b) s'ouvrant à la fois vers le bord interne et le bord externe autour de la forme périphérique, conçues pour permettre différents taux de courbure le long de segments discrets du noyau interne (100) lorsque la force de dilatation interne est introduite à l'intérieur de l'anneau d'annuloplastie (20) et les fentes radiales étant positionnées afin de pousser la forme périphérique en forme de D vers une forme plus circulaire lors de la dilatation.

2. Anneau d'annuloplastie (20) selon la revendication 1, le noyau interne (22 ; 70 ; 80) présentant une première région radialement épaisse (50) au moins le long du côté postérieur (32 ; 74 ; 84) par rapport à des côtés latéraux (34 ; 76 ; 86) adjacents, éventuellement, la première région radialement épaisse (50) possédant environ deux fois l'épaisseur radiale des côtés latéraux (34 ; 76 ; 86).

3. Anneau d'annuloplastie (20) selon la revendication 2, le noyau interne (22 ; 70 ; 80) présentant une seconde région radialement épaisse (52) le long du côté antérieur (30 ; 72 ; 82) par rapport à des côtés latéraux (34 ; 76 ; 86) adjacents, éventuellement, les première et seconde régions radialement épaisses (50, 52) possédant environ deux fois l'épaisseur radiale des côtés latéraux (34 ; 76 ; 86).

4. Anneau d'annuloplastie (20) selon la revendication 3, la seconde région radialement épaisse (52) présentant une pluralité d'encoches (78) écartées, s'ouvrant radialement vers l'extérieur, formées en son sein.

5. Anneau d'annuloplastie (20) selon la revendication 1, possédant une première pluralité des fentes radiales (102a) s'ouvrant vers l'extérieur et espacées le long du côté antérieur (104), éventuellement, possédant une seconde pluralité des fentes radiales (102b) s'ouvrant vers l'extérieur et espacées le long du côté postérieur (106).

6. Anneau d'annuloplastie (20) selon la revendication 1 ou 5, une pluralité de fentes radiales (102a, 102b) s'ouvrant vers l'intérieur et étant espacées le long des côtés latéraux (108).

7. Anneau d'annuloplastie (20) selon l'une quelconque des revendications 1, 5 et 6, chacune des fentes radiales (102a, 102b) étant formée par un canal (116) sensiblement droit s'étendant radialement à partir du bord respectif du noyau interne (100) à une zone en relief (118) fermée agrandie, éventuellement, chacune des zones en relief (118) fermées étant circulaire.

8. Anneau d'annuloplastie (20) selon l'une quelconque des revendications 1 à 7, la forme périphérique étant asymétrique à travers le petit axe (44 ; 114).

9. Anneau d'annuloplastie (20) selon la revendication 8, la forme périphérique étant tridimensionnelle et s'inclinant sur un côté du petit axe (44 ; 114) depuis le côté antérieur (30 ; 72 ; 82 ; 104).

10. Anneau d'annuloplastie (20) selon l'une quelconque des revendications 1 à 9, le noyau interne (22 ; 70 ; 80 ; 100) étant formé avec une section transversale solide et constitué d'un métal semi-rigide.

11. Anneau d'annuloplastie (20) selon la revendication 10, le métal semi-rigide étant choisi dans le groupe constitué par le nitinol, le cobalt-chrome, l'acier inoxydable et un alliage de titane.

12. Anneau d'annuloplastie (20) selon l'une quelconque des revendications 1 à 9, comportant en outre au moins un joint de dilatation (142 ; 172) autour de la forme périphérique le long du noyau interne qui permet à une circonférence du noyau interne de se dilater lorsque celui-ci est soumis à la force de dilatation interne.

13. Combinaison d'un anneau d'annuloplastie (20) pour le remodelage de la valvule mitrale conçu pour une procédure "valve-in-ring" et d'une valvule cardiaque prothétique (60) déployable, comprenant :
un anneau d'annuloplastie (20) selon l'une quelconque des revendications 1 à 12 ; et
une valvule cardiaque prothétique (60) déployable présentant un cadre support déployable conçu pour se déployer plastiquement présentant une section transversale généralement circulaire lorsqu'il est soumis à une force de dilatation vers l'extérieur, le cadre support étant recouvert par un revêtement en tissu entouré par un manchon d'étanchéité (120) fixé formé d'un matériau compressible ou non élastique, la valvule cardiaque (60) présentant une forme contractée pour l'avancement à l'intérieur de l'anneau d'annuloplastie (20) et une forme déployée dimensionnée pour entrer en contact et dilater l'anneau d'annuloplastie (20) et convertir la forme périphérique en une forme plus circulaire, le manchon d'étanchéité (120) s'étendant dans des interstices (132) entre la valvule cardiaque (60) et l'anneau d'annuloplastie (20) et étant conçu pour former des régions radialement plus épaisses et plus minces autour d'une circonférence de la valvule cardiaque (60), éventuellement, le manchon d'étanchéité (120) comprenant un matériau solide qui est plastiquement déformable.

14. Combinaison selon la revendication 13, le manchon d'étanchéité (120) se fixant au revêtement en tissu le long d'une ligne circonférentielle de façon à découpler un raccourcissement axial du cadre support déployable du manchon d'étanchéité (120).

15. Combinaison selon la revendication 13 ou 14, le manchon d'étanchéité (120) comprenant une poche annulaire présentant des parois interne et externe contenant un fluide en son sein, éventuellement, la poche annulaire étant en poly(téréphtalate d'éthylène) et le fluide étant de la silicone.
